(19) Europäisches Patentamt
European Patent Office
Office européen des brevets

(11) Numéro de publication: **0 248 736 B1**

(12) **FASCICULE DE BREVET EUROPEEN**

(45) Date de publication du fascicule du brevet: **28.11.90**

(51) Int. Cl.[5]: **C07D 417/12**, A61K 31/425, A61K 31/415

(21) Numéro de dépôt: **87401254.5**

(22) Date de dépôt: **04.06.87**

(54) **Nouveaux dérivés du benzimidazolylthiométhyl benzothiazole substitué et leurs sels, procédé de prépration, application à titre de médicaments et compositions les renfermant.**

(30) Priorité: **04.06.86 GB 8613592**

(43) Date de publication de la demande: **09.12.87 Bulletin 87/50**

(45) Mention de la délivrance du brevet: **28.11.90 Bulletin 90/48**

(84) Etats contractants désignés: **CH DE FR IT LI NL**

(56) Documents cités: **EP-A- 0 178 438**

(73) Titulaire: **ROUSSEL-UCLAF, 35, boulevard des Invalides, F-75007 Paris(FR)**

(72) Inventeur: **Clements-Jewery Stephen, 14 Church Walk Ashton Keynes, Nr Swindon Wiltshire(GB)**
Inventeur: **Kennewell, Peter David, 10 St Helen's View Okus, Swindon Wiltshire(GB)**
Inventeur: **Westwood, Robert, Martagon 6 Rimes Close, Kingston Bagpuize Oxfordshire(GB)**

(74) Mandataire: **Vieillefosse, Jean-Claude et al, Département des Brevets ROUSSEL UCLAF B.P no 9, F-93230 Romainville(FR)**

**EP 0 248 736 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés du benzimidazolylthiométhyl benzothiazole substitué, ainsi que leurs sels, le procédé de préparation et l'application à titre de médicament de ces nouveaux produits.

L'invention a pour objet de nouveaux dérivés du benzimidazolylthiométhyl benzothiazole substitué, caractérisés en ce qu'ils répondent à la formule générale (I) :

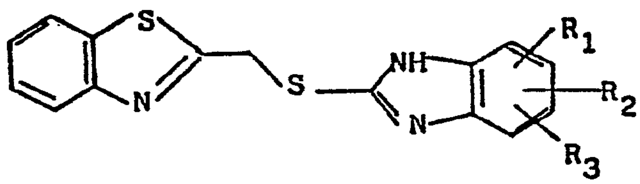

(I)

dans laquelle $R_1$, $R_2$ et $R_3$, identiques ou différents, représentent un atome d'hydrogène, un atome d'halogène, un radical alcoyle renfermant de 1 à 6 atomes de carbone ou un radical alcoxy renfermant de 1 à 6 atomes de carbone, ainsi que leurs sels d'addition avec les acides.

Dans la formule générale (I) et dans ce qui suit, le terme radical alcoyle renfermant de 1 à 6 atomes de carbone désigne par exemple un radical méthyle, éthyle, propyle, isopropyle, butyle, isobutyle, pentyle ou hexyle ;
le terme radical alcoxy renfermant de 1 à 6 atomes de carbone désigne, par exemple, un radical méthoxy, éthoxy, propoxy, isopropoxy, butoxy, pentoxy ou hexyloxy;
le terme atome d'halogène désigne de préférence un atome de chlore ou de brome.

Les sels d'addition avec les acides minéraux ou organiques peuvent être, par exemple, les sels formés avec les acides chlorhydrique, bromhydrique, iodhydrique, nitrique, sulfurique, phosphorique, formique, benzoïque, maléique, fumarique, succinique, tartrique, citrique, oxalique, glyoxylique, aspartique, alcane sulfoniques, tels que l'acide méthane sulfonique et aryl sulfoniques, tels que l'acide benzène sulfonique.

Parmi les produits objet de l'invention, on retient plus particulièrement les dérivés répondant à la formule (I) ci-dessus, ainsi que leurs sels d'addition avec les acides, caractérisés en ce que dans la dite formule (I), $R_1$, $R_2$ et $R_3$, identiques ou différents, représentent un atome d'hydrogène, un atome de chlore, un radical méthyle ou un radical méthoxy.

Parmi ceux-ci, on retient plus particulièrement les dérivés répondant à la formule (I) ci-dessus ainsi que leurs sels d'addition avec les acides, caractérisés en ce que dans ladite formule (I), $R_1$, $R_2$ et $R_3$ identiques ou différents, représentent un atome d'hydrogène ou un atome de chlore.

Parmi ces derniers, on peut citer tout particulièrement les dérivés du benzimidazolylthiométhyl benzothiazole substitué dont les noms suivent :
- le 2-(5,6-dichloro-1H-benzimidazol-2-ylthiométhyl) benzothiazole ;
- le 2-(5-chloro-1H-benzimidazol-2-ylthiométhyl) benzothiazole ;
- le 2-(1H-benzimidazol-2-ylthiométhyl) benzothiazole,

ainsi que leurs sels d'addition avec les acides.

L'invention a également pour objet un procédé de préparation des nouveaux dérivés du benzimidazolylthiométhyl benzothiazole substitué tels que définis ci-dessus, ainsi que leurs sels d'addition avec les acides, caractérisé en ce que l'on fait réagir un produit de formule (II) :

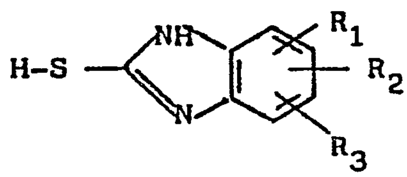

(II)

sous forme d'un sel, dans laquelle $R_1$, $R_2$ et $R_3$ ont la signification déjà indiquée, avec un produit de formule (III) :

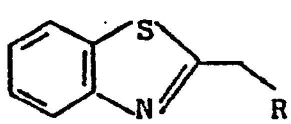

(III)

dans laquelle R représente un atome d'halogène tel qu'un atome de chlore, de brome pu d'iode pour obtenir un produit de formule (I) que l'on isole et si désiré salifie.

Dans des conditions préférentielles de mise en oeuvre de l'invention, le procédé de préparation ci-dessus décrit est caractérisé en ce que :
- la réaction du produit de formule (II) sous forme de sel, avec le produit de formule (III) est effectuée au sein d'un solvant organique tel que l'éther éthylique, le tétrahydrofuranne ou le diméthylformamide ;
- le sel du produit de formule (II) peut être préparé en traitant un produit de formule (II) par un réactif capable de former un anion. Un tel réactif est de préférence un hydrure de métal alcalin tel que l'hydrure de sodium.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objets de la présente invention, possèdent de très intéressantes propriétés pharmacologiques ; ils sont doués notamment de remarquables propriétés inhibitrices de la 5-lipoxygénase et de la liaison de la leukotriène $D_4$ à ses récepteurs. En tant que tels, ils peuvent être utilisés pour leurs propriétés anti-allergiques.

Ces propriétés justifient l'utilisation des nouveaux dérivés de formule (I), objet de la présente l'invention, ainsi que de leurs sels pharmaceutiquement acceptables, à titre de médicaments.

La présente demande a ainsi également pour objet l'application à titre de médicaments des nouveaux dérivés tels que définis par la formule (I), ainsi que de leurs sels d'addition avec les acides pharmaceutiquement acceptables.

Parmi les médicaments objet de l'invention, on retient de préférence les médicaments caractérisés en ce qu'ils sont constitués par les dérivés répondant à la formule (I) dans laquelle $R_1$, $R_2$ et $R_3$ identiques ou différents représentent un atome d'hydrogène, un atome de chlore, un radical méthyle ou un radical méthoxy, ainsi que par leurs sels d'addition avec les acides pharmaceutiquement acceptables.

Parmi ceux-ci, on retient notamment les médicaments caractérisés en ce qu'ils sont constitués par les dérivés répondant à la formule (I) dans laquelle $R_1$, $R_2$ et $R_3$ identiques ou différents représentent un atome d'hydrogène ou un atome de chlore.

Parmi ces derniers, on retient tout particulièrement les produits dont les noms suivent :
- le 2-(5,6-dichloro-1H-benzimidazol-2-ylthiométhyl) benzothiazole ;
- le 2-(5-chloro-1H-benzimidazol-2-ylthiométhyl) benzothiazole ;
- le 2-(1H-benzimidazol-2-ylthiométhyl) benzothiazole,

ainsi que leurs sels d'addition avec les acides pharmaceutiquement acceptables.

Ces médicaments trouvent, par exemple, leur emploi dans le traitement de l'asthme allergique et des bronchites asthmatiformes d'origine allergique et inflammatoire.

La dose usuelle, variable selon le produit utilisé, le sujet traité et l'affection en cause peut être, par exemple, de 0,1 mg à 200 mg par jour, par voie orale chez l'homme.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être par exemple, solides ou liquides et se présenter sous les formes pharmaceutiques couramment utilisées en médecine humaine, comme, par exemple, les comprimés, simples ou dragéifiés, les gélules, les granulés, les suppositoires, les sirops, les aérosols, les crèmes, les pommades, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples suivants illustrent l'invention sans toutefois la limiter.

Exemple 1: 2-(1H-benzimidazol-2-ylthiométhyl) benzothiazole.

On ajoute à 4°C sous atmosphère inerte 1,5 g d'hydrure de sodium dans une solution de 7,6 g de 2-benzimidazolthiol dans 76 cm3 de diméthylformamide. On agite pendant 2 heures à température ambiante, élimine le solvant sous pression réduite, reprend le résidu dans l'acétate d'éthyle, lave avec une solution aqueuse d'hydroxyde de sodium (2N) glacée puis une solution aqueuse saturée en chlorure de sodium, sèche et élimine le solvant sous pression réduite. On obtient 6,91 g de produit attendu après cristallisation dans le dichlorométhane.

Exemples 2 et 3 :

En utilisant une méthode analogue à celle décrite à l'exemple 1 mais en partant des composés correspondants de formule (II) dans laquelle $R_1$, $R_2$ et $R_3$ ont les significations indiquées dans le tableau I ci-après, on a préparé les produits des exemples 2 et 3 (voir tableau I ci-après). La microanalyse, les analyses spectrométriques, les rendements et les points de fusion des composés sont aussi donnés dans le tableau I.

Exemple 2 : 2-(5-chloro-1H-benzimidazol-2-ylthiométhyl) benzothiazole.

Exemple 3 : 2-(5,6-dichloro-1H-benzimidazol-2-ylthiométhyl) benzothiazole.

TABLEAU I

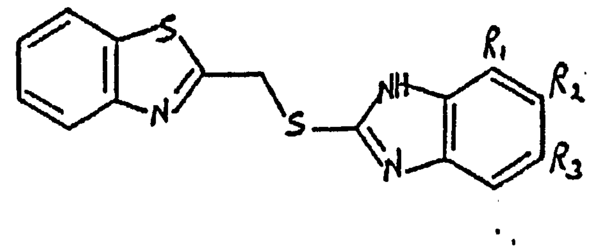

| Exemple | $R_1$ | $R_2$ | $R_3$ | Rendement | F °C | Formule | Microanalyse Calculé/Trouvé C | H | N |
|---|---|---|---|---|---|---|---|---|---|
| 1 | H | H | H | 58 | 159-62 | $C_{15}H_{11}N_3S_2$ | 60.58<br>60.37 | 3.73<br>3.73 | 14.13<br>14.11 |
| 2 | H | Cl | H | 33 | 176-80 | $C_{15}H_{10}ClN_3S_2$ | 54.29<br>54.03 | 3.04<br>3.12 | 12.66<br>12.54 |
| 3 | H | Cl | Cl | 30 | 209-10 (decomp) | $C_{15}H_9Cl_2N_3S_2$ | 49.19<br>49.07 | 2.48<br>2.60 | 11.47<br>11.38 |

Exemple 4 :

On a préparé des comprimés répondant à la formulation suivante :
- Produit de l'exemple 3 .................................... 10 mg
- Excipient q.s. pour un comprimé terminé à ................. 100 mg.
(Détail de l'excipient : lactose, amidon, talc, stéarate de magnésium).

Exemple 5 :

On a préparé des comprimés répondant à la formulation suivante :
- Produit de l'exemple 2 .................................... 10 mg.
- Excipient q.s. pour un comprimé terminé à ................. 100 mg.
(Détail de l'excipient : lactose, amidon, talc, stéarate de magnésium).

Exemple 6 :

On a préparé un aérosol délivrant par dose :
- Produit de l'exemple 3 .................................... 1 mg
- Emulsifiant ............................................... 0,15 mg
- Propulseur ................................................ 50 mg.

ETUDE BIOCHIMIQUE

5-Lipox

L'inhibition de l'ionophore $Ca^{++}$ (A 23187) provoque la libération de produits 5-lipoxygénase (Leukotriene $B_4$ et 5-HETE) de l'acide /14C/ arachidonique des neutrophiles péritonéales pré-marqués du rat.

On a utilisé la méthode de Ahnfelt-Ronne, I. et Arrigoni-Martelli, E. Biochemical Pharmacology, Vol. 31, N⌧ 16, p. 2619-2624 (1982) modifiée. Les valeurs indiquées sont des concentrations micromolaires du composé testé provoquant 50% d'inhibition par rapport à la réponse du témoin déterminées graphiquement d'après les courbes de réponse par dose.

Les résultats de ces tests sont donnés dans le tableau II.

TABLEAU TT

| Exemple | 5-Lipox |
|---|---|
| 1 | 3,5 |
| 2 | 0,59 |
| 3 | 0,23 |

**Revendications**

1) Dérivés du benzimidazolylthiométhyl benzothiazole substitué ainsi que leurs sels d'addition avec les acides, caractérisés en ce qu'ils répondent à la formule générale (I) :

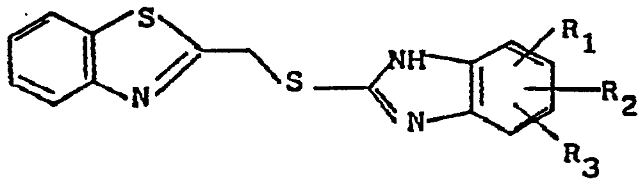

(I)

dans laquelle $R_1$, $R_2$ et $R_3$, identiques ou différents, représentent un atome d'hydrogène, un atome d'halogène, un radical alcoyle renfermant de 1 à 6 atomes de carbone ou un radical alcoxy renfermant de 1 à 6 atomes de carbone.

2) Dérivés du benzimidazolylthiométhyl benzothiazole substitué de formule (I) telle que définie à la revendication 1, ainsi que leurs sels d'addition avec les acides, caractérisés en ce que dans ladite formule (I), $R_1$, $R_2$ et $R_3$ identiques ou différents, représentent un atome d'hydrogène ou un atome de chlore, un radical méthyl ou un radical méthoxy.

3) Dérivés du benzimidazolylthiométhyl benzothiazole substitué de formule (I) telle que définie à la revendication 1 ou 2, ainsi que leurs sels d'addition avec les acides, caractérisés en ce que dans ladite formule (I), $R_1$, $R_2$ et $R_3$ identiques ou différents, représentent un atome d'hydrogène ou un atome de chlore.

4) L'un quelconque des produits de formule (I) telle que définie à la revendication 1, 2 ou 3, dont les noms suivent :
- le 2-(5,6-dichloro-1H-benzimidazol-2-ylthiométhyl) benzothiazole ;
- le 2-(5-chloro-1H-benzimidazol-2-ylthiométhyl) benzothiazole ;
- le 2-(1H-benzimidazol-2-ylthiométhyl) benzothiazole,

ainsi que leurs sels d'addition avec les acides.

5) Procédé de préparation des dérivés du benzimidazolylthiométhyl benzothiazole substitué ainsi que de leurs sels d'addition avec les acides, caractérisé en ce que l'on fait réagir un produit de formule (II) :

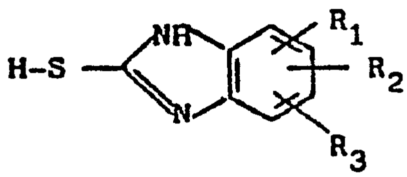

(II)

sous forme d'un sel, dans laquelle $R_1$, $R_2$ et $R_3$ ont la signification déjà indiquée, avec un produit de formule (III) :

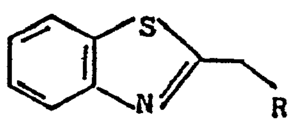

(III)

dans laquelle R représente un atome d'halogène tel qu'un atome de chlore, de brome ou d'iode, pour obtenir un produit de formule (I) que l'on isole et si désiré salifie.

6) Procédé de préparation des dérivés du benzimidazolylthiométhyl benzothiazole substitué tel que défini à la revendication 5, caractérisé en ce que :
- la réaction du produit de formule (III) sous forme de sel avec le produit de formule (III) est effectué au sein d'un solvant organique ;
- le sel du produit de formule (II) peut être préparé en traitant un produit de formule (II) par un réactif capable de former un anion, tel qu'un hydrure de métal alcalin.

7) Médicaments, caractérisés en ce qu'ils sont contitués par lesdérivés du benzimidazolylthiométhyl benzothiazole substitué tels que définis par la formule (I) de la revendication 1 ainsi que par leurs sels d'addition avec les acides pharmaceutiquement acceptables.

8) Médicaments, caractérisés en ce qu'ils sont constitués par les dérivés tels que définis à l'une quelconque des revendications 2 à 4, ainsi que par leurs sels d'addition avec les acides pharmaceutiquement acceptables.

9) Compositions pharmaceutiques, caractérisées en ce qu'elles renferment à titre de principe actif, l'un au moins des médicaments tels que définis à l'une quelconque des revendications 7 ou 8.

**Patentansprüche**

1. Substituierte Benzimidazolylthiomethyl-benzothiazol-Derivate sowie deren Additionssalze mit Säuren, dadurch gekennzeichnet, daß sie der allgemeinen Formel (I)

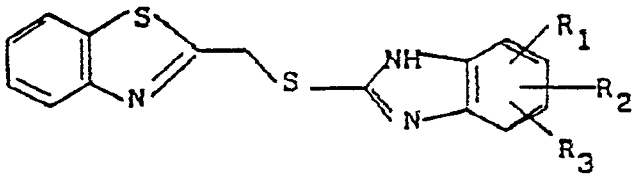

(I)

entsprechen, worin $R_1$, $R_2$ und $R_3$, die gleich oder voneinander verschieden sind, für ein Wasserstoffatom, ein Halogenatom, einen Alkylrest mit 1 bis 6 Kohlenstoffatomen oder einen Alkoxyrest mit 1 bis 6 Kohlenstoffatomen stehen.

2. Substituierte Benzimidazolylthiomethyl-benzothiazol-Derivate der Formel (I) gemäß Anspruch 1 sowie deren Additionssalze mit Säuren, dadurch gekennzeichnet, daß in der Formel (I) $R_1$, $R_2$ und $R_3$, die gleich oder voneinander verschieden sind, für ein Wasserstoffatom oder ein Chloratom, einen Methylrest oder einen Methoxyrest stehen.

3. Substituierte Benzimidazolylthiomethyl-benzothiazol-Derivate der Formel (I) gemäß Anspruch 1 oder 2 sowie deren Additionssalze mit Säuren, dadurch gekennzeichnet, daß in der Formel (I) $R_1$, $R_2$ und $R_3$, die gleich oder voneinander verschieden sind, für ein Wasserstoffatom oder ein Chloratom stehen.

4. Eines der Produkte der Formel (I) gemäß Anspruch 1, 2 oder 3 mit den folgenden Bezeichnungen:
2-(5,6-Dichlor-1H-benzimidazol-2-yl-thiomethyl)benzothiazol;
2-(5-Chlor-1H-benzimidazol-2-yl-thiomethyl)-benzothiazol;
2-(1H-Benzimidazol-2-yl-thiometyl)-benzothiazol sowie deren Additionssalze mit Säuren.

5. Verfahren zur Herstellung von substituierten Benzimidazolylthiomethyl-benzothiazol-Derivaten sowie deren Additionssalzen mit Säuren, dadurch gekennzeichnet, daß man ein Produkt der Formel (II)

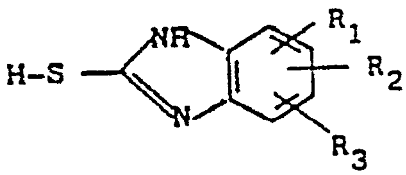

(II)

in Form eines Salzes, worin $R_1$, $R_2$ und $R_3$ die angegebene Bedeutung besitzen, mit einem Produkt der Formel (III)

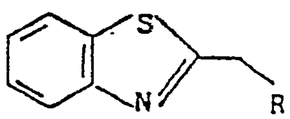

(III)

worin R für ein Halogenatom, wie ein Chlor-, Brom- oder Jodatom, steht, umsetzt, um zu einem Produkt der Formel (I) zu gelangen, das man isoliert und gewünschtenfalls in ein Salz überführt.

6. Verfahren zur Herstellung der substituierten Benzimidazolylthiomethyl-benzothiazol-Derivate, wie in Anspruch 5 definiert, dadurch gekennzeichnet, daß die Umsetzung des Produkts der Formel (II) in Form des Salzes mit dem Produkt der Formel (III) in dem Medium eines organischen Lösungsmittels erfolgt, das Salz des Produkts der Formel (II) hergestellt werden kann, indem man ein Produkt der Formel (II) mit einem Reagens, das zur Bildung eines Anions befähigt ist, wie einem Alkalimetallhydrid, behandelt.

7. Arzneimittel, dadurch gekennzeichnet, daß sie aus den substituierten Benzimidazolylthiomethyl-benzothiazol-Derivaten der Formel (I) gemäß Anspruch 1 oder aus deren Additionssalzen mit pharmazeutisch verträglichen Säuren bestehen.

8. Arzneimittel, dadurch gekennzeichnet, daß sie aus den Derivaten gemäß einem der Ansprüche 2 bis 4 oder aus deren Additionssalzen mit pharmazeutisch verträglichen Säuren bestehen.

9. Pharmazeutische Zusammensetzungen, dadurch gekennzeichnet, daß sie als Wirkstoff zumindest eines der Arzneimittel gemäß einem der Ansprüche 7 oder 8 enthalten.

**Claims**

1. Derivatives of substituted benzimidazolylthiomethyl benzothiazole as well as their addition salts with acids, characterised in that they correspond to the general formula (I):

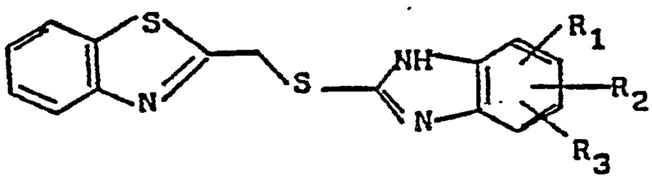

(I)

in which $R_1$, $R_2$ and $R_3$, identical or different, represent a hydrogen atom, a halogen atom, an alkyl radical containing 1 to 6 carbon atoms or an alkoxy radical containing 1 to 6 carbon atoms.

2. Derivatives of substituted benzimidazolylthiomethyl benzothiazole of formula (I) as defined in claim 1, as well as their addition salts with acids, characterised in that in said formula (I), $R_1$, $R_2$ and $R_3$, identical or different, represent a hydrogen atom or a chlorine atom, a methyl radical or a methoxy radical.

3. Derivatives of substituted benzimidazolylthiomethyl benzothiazole of formula (I) as defined in claim 1 or 2, as well as their addition salts with acids, characterised in that in said formula (I), $R_1$, $R_2$ and $R_3$, identical or different, represent a hydrogen atom or a chlorine atom.

4. Any one of the products of formula (I) as defined in claim 1, 2 or 3, the names of which follow:
– 2-(5,6-dichloro-1H-benzimidazol-2-ylthiomethyl) benzothiazole;
– 2-(5-chloro-1H-benzimidazol-2-ylthiomethyl) benzothiazole;
– 2-(1H-benzimidazol-2-ylthiomethyl) benzothiazole, as well as their addition salts with acids.

5. Preparation process of derivatives of substituted benzimidazolylthiomethyl benzothiazole as well as their addition salts with acids, characterised in that a product of formula (II):

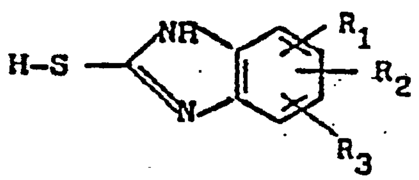

(II)

in the form of a salt, in which $R_1$, $R_2$ and $R_3$ have the meaning already indicated, is reacted with a product of formula (III):

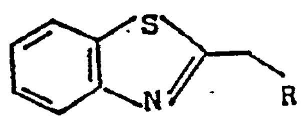

(III)

in which R represents a halogen atom such as chlorine, bromine or iodine, so as to obtain a product of formula (I) which is isolated and if desired salified.

6. Preparation process of derivatives of substituted benzimidazolylthiomethyl benzothiazole as defined in claim 5, characterised in that:
– the reaction of the product of formula (III) in the form of a salt with the product of formula (III) is carried out in an organic solvent;
– the salt of the product of formula (II) can be prepared by treating a product of formula (II) with a reagent capable of forming an anion, such as an alkali metal hydride.

7. Medicaments, characterised in that they are constituted by derivatives of substituted benzimidazolylthiomethyl benzothiazole as defined by formula (I) in claim 1 as well as their addition salts with pharmaceutically acceptable acids.

8. Medicaments, characterised in that they are constituted by the derivatives as defined in any one of claims 2 to 4, as well as their addition salts with pharmaceutically acceptable acids.

9. Pharmaceutical compositions, characterised in that they contain as active ingredient, at least one of the medicaments as defined in any one of claims 7 or 8.